# EUROPÄISCHE PATENTANMELDUNG

(11) **EP 3 415 115 A1**
(43) Veröffentlichungstag der Anmeldung: **19.12.2018**
(21) Anmeldenummer: 17175644.8
(22) Anmeldetag: 13.06.2017
(51) Int. Cl.: A61C 19/00, A61B 50/20

(54) **TRÄGERVORRICHTUNG FÜR EIN AUFZUBEREITENDES, DENTALES INSTRUMENT**

(71) Anmelder: W & H Dentalwerk Bürmoos GmbH, 5111 Bürmoos (AT)
(72) Erfinder: LETTE, Andreas, 4050 Traun (AT); EIBL, Johann, 5230 Mattighofen (AT)
(74) Vertreter: Benda, Ralf

(57) **Zusammenfassung**

Trägervorrichtung (1) für zumindest ein aufzubereitendes, dentales Instrument (2 - 5), umfassend: eine Seitenwand-lose Gitterplatte (6), deren Außenumfang durch einen Rahmen (7) mit vier separaten, sich gerade erstreckenden Seiten (8A, 8B) begrenzt ist, wobei die vier Seiten (8A, 8B) des Rahmens (7) durch abgerundete Rahmenabschnitte (9) miteinander verbunden sind, ein von dem Rahmen (7) umschlossenes Gitter (10) und zumindest ein an dem Gitter (10) befestigtes oder befestigbares und von dem Gitter (10) abstehendes Lagerelement (11 - 13), an dem das aufzubereitende Instrument (2 - 5) lagerbar ist. Ein Aufbereitungsverfahren für ein dentales Instrument (2 - 5), bei dem das auf einer entsprechenden Trägervorrichtung (1) angeordnete dentale Instrument (2 - 5) zuerst in ein Reinigungs- und/ oder Desinfektionsgerät und anschließend in eine Sterilisationsvorrichtung eingebracht wird, ist ebenfalls beschrieben.

## Beschreibung

Die vorliegende Erfindung betrifft eine Trägervorrichtung für zumindest ein aufzubereitendes, d.h. zu reinigendes, desinfizierendes und/ oder zu sterilisierendes, dentales Instrument, auf der das Instrument befestigt und anschließend in ein Reinigungsund/ oder Desinfektionsgerät und in eine Sterilisationsvorrichtung geben werden kann.

Bekanntermaßen sind dentale, insbesondere dentalchirurgische Instrumente nach ihrer Verwendung bzw. vor ihrer nächsten Verwendung aufzubereiten. Eine derartige Aufbereitung umfasst in einem ersten Schritt das maschinelle Reinigen oder Desinfizieren in einem Reinigungs- und/ oder Desinfektionsgerät (oftmals mit "RDG" abgekürzt) und anschließend in einem zweiten Schritt das Sterilisieren in einer Sterilisationsvorrichtung.

Üblicherweise wird ein aufzubereitendes dentales Instrument für das Reinigen oder Desinfizieren in einem Reinigungs- und/ oder Desinfektionsgerät in einer Gitter- oder Drahtbox gelagert. Nach der Reinigung und/ oder Desinfektion und dem Trocknen wird das dentale Instrument aus der Gitter- oder Drahtbox entnommen und in einem Sterilbeutel verpackt, vorzugsweise in eine Kassette platziert, die anschließend in einen Sterilbeutel gesteckt wird. Anschließend wird das dentale Instrument mit dem Sterilbeutel in die Sterilisationsvorrichtung gegeben, sterilisiert und anschließend in dem Sterilbeutel bis zur erneuten Verwendung steril gelagert.

Dieses beschrieben Verfahren, insbesondere das zweimalige Verpacken des dentalen Instruments zuerst in der Gitter- oder Drahtbox und anschließend in dem Sterilbeutel, ist umständlich und zeitaufwendig. Aus diesem Grund wurde bereits vorgeschlagen, das aufzubereitende, dentale Instrument in einer Kassette zu lagern, die in einem Reinigungs- und/ oder Desinfektionsgerät und in einer Sterilisationsvorrichtung verwendbar ist, sodass das Umpacken des aufzubereitenden, dentalen Instruments nach dem Reinigen oder Desinfizieren von der Gitter- oder Drahtbox in den Sterilbeutel entfällt, siehe DE 20 2016 005 828 U1.

Der in der DE 20 2016 005 828 U1 beschriebene Behälter zur Wiederaufbereitung von medizinischen Instrumenten, insbesondere hohler Dentalinstrumente, besteht aus einer Unterschale und einem Deckel, der durch eine Dichtung die innere Atmosphäre von der äußeren trennt. Der Behälter umfasst auch Adapteranschlüsse, durch welche Reinigungsflüssigkeiten, Desinfektionsflüssigkeiten, Gase und Öle gepumpt werden, um die Instrumente innerlich aufzubereiten und zu pflegen.

Der genannte Behälter entspricht in seinem Aufbau im Wesentlichen bekannten Kassetten für Sterilisationsvorrichtungen. Er ist jedoch aufgrund seines Aufbaus für den Einsatz in einem Reinigungs- und/ oder Desinfektionsgerät wenig geeignet. Reinigungs- und/ oder Desinfektionsgeräte ähneln in ihrem Aufbau Geschirrspülmaschinen und haben wie diese Gitterkörbe mit abstehenden Drähten und Drahtgestellen, an denen die zu reinigenden Instrumente gelagert werden können. Der in der DE 20 2016 005 828 U1 beschriebene Behälter ist in einem derartigen Drahtgestell schlecht platzierbar bzw. es können demgemäß nur wenige dieser Behälter in einem Gitterkorb angeordnet werden.

Ein weiterer Nachteil des in der DE 20 2016 005 828 U1 offenbarten Behälters besteht in seinem geschlossenen, kassettenartigen Aufbau, der ein gründliches Reinigen und/ oder Desinfizieren der Außenseite des dentalen Instruments behindert. So gelangt insbesondere die von Düsen des Reinigungs- und/ oder Desinfektionsgeräts abgegebene Flüssigkeit nicht zu dem aufzubereitenden dentalen Instrument.

Der vorliegenden Erfindung liegt daher die Aufgabe zugrunde eine verbesserte Trägervorrichtung zur Verwendung in einem Reinigungs- und/ oder Desinfektionsgerät und in einer Sterilisationsvorrichtung für zumindest ein aufzubereitendes, d.h. zu reinigendes, desinfizierendes und/ oder zu sterilisierendes, dentales Instrument zu schaffen. Die Trägervorrichtung soll insbesondere derart beschaffen sein, dass sie platzsparend in einem Reinigungs- und/ oder Desinfektionsgerät anordenbar ist und eine möglichst umfassende Reinigung und/ oder Desinfektion in einem Reinigungs- und/ oder Desinfektionsgerät sowie eine möglichst optimale Sterilisation in einer Sterilisationsvorrichtung zulässt.

Diese Aufgabe wird gemäß der vorliegenden Erfindung durch eine Trägervorrichtung für zumindest ein aufzubereitendes, d.h. zu reinigendes, desinfizierendes und/ oder zu sterilisierendes, dentales Instrument mit den Merkmalen des Anspruchs 1 gelöst. Besonders vorteilhafte Ausführungsformen sind in den Unteransprüchen angeführt. Ein entsprechendes Aufbereitungsverfahren für ein dentales Instrument mit einer derartigen Trägervorrichtung und die Verwendung einer derartigen Trägervorrichtung in einem Aufbereitungsverfahren sind zusätzlich in den Ansprüchen angegeben.

Die Trägervorrichtung für zumindest ein aufzubereitendes, dentales Instrument, umfasst eine seitenwand-lose Gitterplatte, deren Außenumfang durch einen Rahmen mit vier separaten, sich gerade erstreckenden Seiten begrenzt ist, wobei die vier Seiten des Rahmens durch abgerundete Rahmenabschnitte miteinander verbunden sind, ein von dem Rahmen umschlossenes Gitter und zumindest ein an dem Gitter befestigbares und von dem Gitter abstehendes Lagerelement, an dem das aufzubereitende Instrument lagerbar ist.

Die Trägervorrichtung ist aufgrund ihres Aufbaus hervorragend für den Einsatz in einem Reinigungs- und/ oder Desinfektionsgerät und in einer Sterilisationsvorrichtung geeignet. Da die Trägervorrichtung eine seitenwand-lose Gitterplatte aufweist, insbesondere als Basis somit ausschließlich aus einer ebenen Gitterplatte besteht und keine Seitenwände oder einen Deckel hat, benötigt sie sehr wenig Platz und kann damit platzsparend in den Drahtgestellen der Gitterkörbe von Reinigungs- und/ oder Desinfektionsgeräten positioniert werden. Entsprechend kann eine größere Anzahl von Trägervorrichtungen in einem Reinigungs- und/ oder Desinfektionsgeräten angeordnet werden und somit die Anzahl der pro Waschgang gereinigten/ desinfizierten dentalen Instrumente erhöht werden. Die Trägervorrichtung erhöht somit die Wirtschaftlichkeit eines Reinigungs- und/ oder Desinfektionsgeräts.

Aufgrund des Vorsehens der seitenwand-Iosen Gitterplatte und somit des Fehlens von Seitenwänden oder eines Deckels ist das zumindest eine auf der Trägervorrichtung gelagerte, aufzubereitende dentale Instrument sehr gut für eine Reinigungs- und/ oder Desinfektionsflüssigkeit zugänglich, wodurch eine verbesserte Reinigung- und/ oder Desinfektion ermöglicht wird. Auch können Schläuche oder Adapter zur Innenaufbereitung des dentalen Instruments auf einfache Weise an das an der Gitterplatte gelagerte dentale Instrument angeschlossen werden oder auch ein derartiger Schlauch durch das Gitter geführt werden.

Ein weiterer Vorteil der Trägervorrichtung besteht in den abgerundeten Rahmenabschnitten, welche die vier Seiten des Rahmens verbinden. Durch das Vorsehen der abgerundeten Rahmenabschnitte (anstelle von spitzen Ecken oder Kanten) ist die Gefahr, dass ein Sterilbeutel, in dem die Trägervorrichtung aufgenommen ist, beschädigt, zum Beispiel eingerissen, wird, erheblich verringert. Die Trägervorrichtung ermöglicht damit eine zuverlässige, sterile Lagerung des sterilisierten dentalen Instruments.

Schließlich ermöglicht das zumindest eine an dem Gitter der seitenwand-Iosen Gitterplatte befestigbare (und davon auch wieder lösbare) Lagerelement, dass ein Anwender die Position des Lagerelements auf dem Gitter gezielt auswählen und an die Größe und/ oder Form des aufzubereitenden dentalen Instruments anpassen kann. Entsprechend kann er bei Verwendung mehrerer Lagerelemente an einem Gitter auch die Relativpositionen der Lagerelemente und/ oder Abstände zwischen den Lagerelementen an das oder die aufzubereitenden dentalen Instrumente anpassen. Dadurch können die Anzahl der aufbereiteten dentalen Instrumente und somit die Wirtschaftlichkeit der Aufbereitung ebenfalls erhöht werden.

Die vier Seiten des Rahmens sind vorzugsweise in im Wesentlichen viereckiger, insbesondere rechteckiger Form angeordnet. Je zwei der vier Seiten sind vorzugsweise im Wesentlichen parallel zueinander angeordnet. Je zwei der vier Seiten, insbesondere jene, die im Wesentlichen parallel zueinander angeordnet sind, haben vorzugsweise im Wesentlichen dieselbe Länge. Die (anstelle von scharfkantigen Ecken oder Winkeln vorgesehenen) abgerundeten Rahmenabschnitte verbinden vorzugsweise jeweils zwei der vier Seiten, insbesondere jeweils zwei Seiten mit unterschiedlichen Längen; besonders bevorzugt verbinden sie diese zwei Seiten derart, dass diese im Wesentlichen rechtwinkelig zueinander angeordnet sind. Die vier Seiten des Rahmens erstrecken sich vorzugsweise im Wesentlichen in jener Ebene, in der sich das Gitter erstreckt.

Die Trägervorrichtung umfasst eine seitenwand-lose Gitterplatte. Dies bedeutet insbesondere, dass an dem Rahmen der Gitterplatte keine Seitenwand vorgesehen ist und/ oder dass sich von dem Rahmen keine Seitenwand erstreckt. Der Außenumfang des Gitters und/ oder der Gitterplatte weist vorzugsweise eine im Wesentlichen viereckige, insbesondere rechteckige Form auf. Das Gitter und/ oder die Gitterplatte ist/ sind im Wesentlichen plan geformt.

Die abgerundeten Rahmenabschnitte umfassen bevorzugt jeweils zumindest eine Biegung, die in der Ebene, in der sich das Gitter erstreckt, um das Gitter, insbesondere um den Außenumfang und/ oder die Eckbereiche des (im Wesentlichen viereckigen) Gitters, verläuft. Das Gitter und/ oder der Gitterplatte, die vier Seiten des Rahmens und die abgerundeten Rahmenabschnitte, insbesondere deren Biegungen, erstrecken sich besonders bevorzugt im Wesentlichen alle in derselben Ebene.

Das Gitter ist vorzugsweise durch eine Vielzahl von einander kreuzenden, besonders bevorzugt im Wesentlichen rechtwinkelig zueinander angeordneten, Gitterstäben oder Gittersträngen gebildet. Die (im rechten Winkel zur Ebene, in der sich das Gitter erstreckt, gemessene) Höhe der Gitterstränge beträgt zumindest 3 mm, vorzugsweise zwischen 3mm und 20 mm. Der Abstand zwischen zwei Gittersträngen (in der Ebene, in der sich das Gitter erstreckt) liegt zwischen 0,5 mm und 40 mm, vorzugsweise im Bereich von 15mm - 25 mm. Die genannten Abmessungen und Abstände der Gitterstränge bewirken in vorteilhafter Weise eine zuverlässige, insbesondere lösbare, Befestigung des zumindest einen Lagerelements an der Trägervorrichtung und eine gute Zugänglichkeit für ein Reinigungs-, Desinfektions- und/ oder Sterilisationsmedium auch an jene Seite des aufzubereitenden Instruments, die dem Gitter zugewandt ist.

Die Außenmaße der Trägervorrichtung sind vorzugsweise wie folgt: die Länge der Trägervorrichtung ist geringer als 280 mm, bevorzugt beträgt die Länge in etwa 275 mm; die Breite der Trägervorrichtung ist geringer als 180 mm, bevorzugt beträgt die Länge in etwa 175 mm. Damit passt die Trägervorrichtung in vorteilhafter Weise in ein Tray oder eine Trägerwanne einer dentalen Sterilisationsvorrichtung.

An der erfindungsgemäßen Trägervorrichtung können eine Vielzahl von unterschiedlichen aufzubereitenden, dentalen Instrumenten gelagert oder befestigt werden. Der Begriff aufzubereitendes, dentales Instrument umfasst damit zum Beispiel Handstücke, Winkelstücke, Adapter, Kupplungen, Zubehörteile für Hand- oder Winkelstücke, zum Beispiel Werkzeugwechsler, dentale motorische Antriebe, insbesondere elektrische, pneumatische oder (Ultra)schall-Antriebe, Schläuche, Kabel, Leitungen, insbesondere lösbar oder unlösbar mit einem Hand- oder Winkelstück verbundene Schläuche, Kabel, Leitungen, und Werkzeuge, wie Bohrer, Zahnsteinentfernungswerkzeuge, Reamer, Gewindeschneider etc. Aufgrund der Möglichkeit, Lagerelemente für aufzubereitende, dentale Instrumente lösbar an der Trägervorrichtung befestigen zu können, kann der Anwender in vorteilhafter Weise in Abhängigkeit der Art des aufzubereitenden Instruments ein geeignetes Lagerelement auswählen und dieses reversibel an der Trägervorrichtung befestigen.

Das zumindest eine Lagerelement ist an der Trägervorrichtung, insbesondere lösbar, befestigt. Das zumindest eine Lagerelement ist insbesondere an und/ oder zwischen den Gitterstäben oder Gittersträngen des Gitters befestigt oder befestigbar. Das zumindest eine Lagerelement steht des Weiteren von der Trägervorrichtung oder dem Gitter ab oder erstreckt sich davon, besonders bevorzugt im Wesentlichen im rechten Winkel.

Das zumindest eine Lagerelement ist vorzugsweise innerhalb des Rahmens und/ oder beabstandet von diesem angeordnet. Alternativ ist es auch möglich, das zumindest eine Lagerelement zumindest teilweise an dem Rahmen der Trägervorrichtung, lösbar oder unlösbar, vorzusehen. Beispielsweise kann das zumindest eine Lagerelement an der Innenseite des Rahmens, welche dem Gitter zugewandt ist, befestigt oder befestigbar sein.

Das zumindest eine Lagerelement kann gemäß einem Ausführungsbeispiel lösbar an dem Gitter befestigt sein. Alternativ ist es auch möglich, das zumindest eine Lagerelement unlösbar mit dem Gitter auszubilden oder zu verbinden. Alternativ ist es auch denkbar, mehrere Lagerelemente an einer Trägervorrichtung vorzusehen, wobei zumindest eines dieser mehreren Lagerelemente lösbar und zumindest ein anderes dieser mehreren Lagerelemente unlösbar an dem Gitter vorgesehen ist. Bei Vorhandensein mehrerer Lagerelemente ist es besonders vorteilhaft, zumindest eines dieser mehreren Lagerelemente lösbar an dem Gitter zu befestigen, um ihre Relativabstände oder Positionen verändern zu können, um die Trägervorrichtung an unterschiedliche aufzubereitende dentale Instrumente anpassen zu können.

Vorzugsweise weist das zumindest eine Lagerelement ein Steckelement mit einer Steckaufnahme auf, in welche das aufzubereitende Instrument einsteckbar ist. Das Steckelement und/ oder die Steckaufnahme und/ oder eine Wand der Steckaufnahme, die einen Aufnahmeraum für zumindest ein Teil des aufzubereitenden Instruments begrenzt, ist/sind zum Beispiel hülsenförmig, gebogen, kreissegmentförmig, stufenförmig, schulterförmig, in Form eines Rücksprungs und/ oder in Form eines Einstichs ausgebildet.

Vorzugsweise definiert die Steckaufnahme einen Aufnahmeraum, in dem zumindest ein Teil des aufzubereitenden Instruments aufnehmbar ist. An einer den Aufnahmeraum begrenzenden Wand der Steckaufnahme ist zumindest ein in den Aufnahmeraum ragender Fortsatz, insbesondere eine Noppe vorgesehen, an welchem das aufzubereitende Instrument lagert, so dass es von der den Aufnahmeraum begrenzenden Wand beabstandet ist. Damit wird in vorteilhafter Weise eine Reinigung, Desinfektion und/ oder Sterilisation auch an einem Großteil jenes Bereichs des aufzubereitenden Instruments ermöglicht, der sich in dem Aufnahmeraum der Steckaufnahme befindet. Besonders bevorzugt ist die den Aufnahmeraum begrenzende Wand der Steckaufnahme gebogen oder kreissegmentförmig geformt, wobei an der Wand mehrere, zum Beispiel drei, in den Aufnahmeraum ragende Fortsätze vorgesehen sind.

Vorzugsweise weist die Steckaufnahme zwei Federarme oder zwei Lagerarme auf, zwischen denen der Aufnahmeraum vorgesehen ist. Vorzugsweise formen und/ oder umgeben die zwei Federarme oder Lagerarme zumindest teilweise den Aufnahmeraum, in dem zumindest ein Teil des aufzubereitenden Instruments aufnehmbar ist. Die zwei Federarme oder Lagerarme umfassen vorzugsweise zumindest teilweise die den Aufnahmeraum begrenzende Wand. Die zwei Federarme oder Lagerarme entspringen vorzugsweise einer gemeinsamen Basis. Die zwei Federarme oder Lagerarme sind vorzugsweise gebogen geformt. Die zwei Federarme oder Lagerarme weisen bevorzugt jeweils ein freies Ende auf, wobei besonders bevorzugt aufgrund der gebogenen Form der beiden Federarme oder Lagerarme die freien Enden einander zugewandt sind. Die zwei Federarme oder Lagerarme sind bevorzugt durch einen Einstich oder Rücksprung in einer Wand des Steckelements oder der Steckaufnahme gebildet.

Der Aufnahmeraum und/ oder die Steckaufnahme ist/ sind bevorzugt derart ausgebildet, dass das aufzubereitende dentale Instrument in den Aufnahmeraum schiebbar und/ oder steckbar ist. Das Schieben und/ oder Stecken umfasst wahlweise ein Bewegen des aufzubereitenden dentalen Instruments im Wesentlichen parallel zu der Ebene, in der sich das Gitter erstreckt, im Wesentlichen gewinkelt, insbesondere rechtwinkelig zu der Ebene, in der sich das Gitter erstreckt, oder im Wesentlichen parallel und gewinkelt zu der genannten Ebene.

Vorzugsweise weist das zumindest eine Lagerelement einen vom Gitter abstehenden Wickelkörper mit einer Wickelfläche auf, wobei ein aufzubereitender Schlauch, eine aufzubereitende Leitung oder ein aufzubereitendes Kabel um den Wickelkörper führbar und an dem Wickelkörper, insbesondere an dessen Wickelfläche lagerbar ist. Vorzugsweise kontaktiert der Schlauch, die Leitung oder das Kabel somit die Wickelfläche oder die Wickelfläche ist jener Abschnitt des Wickelkörpers, der zum Kontakt mit dem Schlauch, der Leitung oder dem Kabel vorgesehen ist. Mit Hilfe dieses oder insbesondere mehrere dieser Wickelkörper können in vorteilhafter Weise ein oder mehrere Schläuche, Kabel oder Leitungen an der Trägervorrichtung gelagert oder gehalten werden.

Der Wickelkörper ist vorzugsweise schildförmig und/ oder gebogen und/ oder als Hohlkörper und/ oder als Halbschale ausgebildet. Das zumindest eine, einen Wickelkörper aufweisende Lagerelement kann lösbar oder unlösbar an dem Gitter befestigt sein. Alternativ ist es auch denkbar, zumindest einen ersten Wickelkörper lösbar und zumindest einen zweiten Wickelkörper unlösbar an dem Gitter vorzusehen. Der zumindest eine Wickelkörper ist bevorzugt an der Peripherie oder im äußeren Randbereich des Gitters angeordnet. Der zumindest eine Wickelkörper ist bevorzugt angrenzend an den Rahmen und/ oder diesen kontaktierend an der Trägervorrichtung vorgesehen.

Vorzugsweise ist der gesamte Wickelkörper oder zumindest die Wickelfläche des Wickelkörpers konvex oder gebogen ausgebildet. Alternativ oder zusätzlich weist der Wickelkörper einen konvexen oder gebogenen Überstand auf. Die konvexe oder gebogene Ausformung bewirkt, dass die Kontaktfläche zwischen dem aufzubereitenden Schlauch oder Kabel und der Wickelfläche möglichst gering ist, so dass eine möglichst vollständige Reinigung, Desinfektion oder Sterilisation des Schlauchs oder Kabels erreicht wird.

Vorzugsweise ist die Wickelfläche des Wickelkörpers im Wesentlichen rechtwinkelig zu einer Ebene angeordnet, in welcher sich das Gitter der Trägervorrichtung erstreckt. Damit ist in vorteilhafter Weise eine besonders zuverlässige Lagerung des Schlauchs oder Kabels an der Wickelfläche und (direkt oder indirekt) auf dem Gitter erzielbar.

Vorzugsweise weist der Wickelkörper an seinem freien, vom Gitter entfernten Ende eine Nase oder einen Vorsprung auf, die/ der dazu vorgesehen ist, den um den Wickelkörper gewickelten Schlauch (bzw. das Kabel oder die Leitung) zu fixieren, um insbesondere ein Abgleiten des Schlauchs von dem Wickelkörper zu verhindern.

Gemäß einem Ausführungsbeispiel sind an der Trägervorrichtung und/ oder dem Gitter mehrere Wickelkörper vorgesehen, wobei zumindest einer dieser mehreren Wickelkörper derart angeordnet oder anordenbar ist, dass seine Wickelfläche einer Längsachse der Trägervorrichtung zugewandt ist, während zumindest ein anderer dieser mehreren Wickelkörper derart angeordnet oder anordenbar ist, dass seine Wickelfläche von der Längsachse der Trägervorrichtung abgewandt ist. Wird ein Schlauch, Kabel oder eine Leitung um diese in unterschiedliche Richtungen weisenden Wickelflächen gewickelt, so werden diese in einfacher und zuverlässiger Weise zwischen den Wickelkörpern und Wickelflächen gehalten oder geklemmt. Besonders bevorzugt sind die mehreren Wickelkörper derart angeordnet, dass (bei aufeinander folgenden oder benachbarten Wickelkörpern) jeweils auf einen Wickelkörper, dessen Wickelfläche der Längsachse der Trägervorrichtung zugewandt ist, ein Wickelkörper folgt, dessen Wickelfläche der Längsachse der Trägervorrichtung abgewandt ist.

Besonders bevorzugt sind an der Trägervorrichtung vorgesehen: zumindest ein Lagerelement, das ein Steckelement mit einer Steckaufnahme aufweist, in welche ein aufzubereitendes, dentales Instrument, insbesondere ein motorischer Antrieb, einsteckbar ist, und mehrere Lagerelemente, die jeweils einen Wickelkörper mit einer Wickelfläche aufweisen, so dass ein aufzubereitender Schlauch oder ein aufzubereitendes Kabel oder eine aufzubereitende Leitung, insbesondere des in dem Steckelement eingesteckten, aufzubereitenden Instruments, um die Wickelkörper führbar und an ihren Wickelflächen lagerbar ist. Vorzugsweise ist das zumindest eine Lagerelement, das ein Steckelement mit einer Steckaufnahme aufweist, lösbar an dem Gitter befestigt und sind die mehrere Lagerelemente, die jeweils einen Wickelkörper mit einer Wickelfläche aufweisen, unlösbar an der Trägervorrichtung, insbesondere dem Gitter fixiert. Alternativ ist es auch denkbar, dass zumindest einer der Wickelkörper ebenfalls lösbar an dem Gitter fixiert ist. Vorzugsweise ist das zumindest eine Lagerelement mit dem Steckelement von den mehreren Lagerelementen, die jeweils einen Wickelkörper aufweisen, umgeben und/ oder das zumindest eine Lagerelement mit dem Steckelement ist zwischen zumindest zwei der mehreren Lagerelemente, die jeweils einen Wickelkörper aufweisen, angeordnet.

Vorzugsweise sind das zumindest eine Lagerelement, das ein Steckelement mit einer Steckaufnahme aufweist, und die mehreren Lagerelemente, die jeweils einen Wickelkörper mit einer Wickelfläche aufweisen, derart auf der Trägervorrichtung angeordnet, dass der aufzubereitende Schlauch oder das aufzubereitende Kabel nicht von dem in dem Steckelement eingesteckten, aufzubereitenden Instrument gelöst werden muss. Dazu ist zum Beispiel vorgesehen, dass die mehreren Lagerelemente, die jeweils einen Wickelkörper mit einer Wickelfläche aufweisen, das zumindest eine Lagerelement, das ein Steckelement mit einer Steckaufnahme aufweist, umgeben, zum Beispiel in einer im Wesentlichen kreisförmigen oder elliptischen Form. Alternativ oder zusätzlich können die mehreren Lagerelemente, die jeweils einen Wickelkörper mit einer Wickelfläche aufweisen, zwischen dem Lagerelement, das ein Steckelement mit einer Steckaufnahme aufweist, und einer Seite des Rahmens angeordnet sein. Alternativ oder zusätzlich ist das zumindest eine Lagerelement, das ein Steckelement mit einer Steckaufnahme aufweist näher zur Längsachse der Trägervorrichtung angeordnet als zumindest eines der mehreren Lagerelemente, die jeweils einen Wickelkörper mit einer Wickelfläche aufweisen.

Vorzugsweise weist das von dem Gitter lösbare zumindest eine Lagerelement, insbesondere das zumindest eine Lagerelement, das ein Steckelement mit einer Steckaufnahme umfasst, zur Befestigung an zumindest einem Gitterstrang oder Gitterstab des Gitters eine Klemm-, Rast- und/ oder Steckvorrichtung auf.

Vorzugsweise umfasst die Klemm-, Rast- und/ oder Steckvorrichtung zumindest ein Klemm-, Rast- und/ oder Steckelement, das zumindest einen Gitterstrang des Gitters umgreift oder hintergreift. Damit ist das zumindest eine Lagerelement zuverlässig an dem Gitter befestigbar. Vorzugsweise sind das zumindest eine Klemm-, Rast- und/ oder Steckelement und die Steckaufnahme des zumindest einen Lagerelements unlösbar miteinander verbunden und/ oder entspringen einer gemeinsamen Basis. Vorzugsweise umfasst das zumindest eine Klemm-, Rast- und/ oder Steckelement eine Nase zum Umgreifen oder Hintergreifen des Gitterstrangs.

Das zumindest eine Klemm-, Rast- und/ oder Steckelement weist zum Beispiel ein Federelement auf oder ist durch ein Federelement gebildet. Vorzugsweise ist das Federelement zumindest teilweise zwischen Gittersträngen des Gitters anordenbar und/ oder es ist durch zumindest einen Gitterstrang bewegbar, insbesondere beim Einstecken in das Gitter. Alternativ oder zusätzlich weist die Klemm-, Rast- und/ oder Steckvorrichtung zwei im Wesentlichen parallel zueinander angeordnete, flächige Anschlusselemente auf, an denen jeweils zumindest ein Klemm-, Rast- und/ oder Steckelement vorgesehen ist und die insbesondere zwischen Gittersträngen des Gitters anordenbar sind.

Vorzugsweise umfasst die Klemm-, Rast- und/ oder Steckvorrichtung mehrere Klemm-, Rast- und/ oder Steckelemente, die insbesondere zusammenwirken, um das zumindest eine Lagerelement am Gitter zu befestigen, und/ oder zwischen denen ein Gitterstrang des Gitters anordenbar und/ oder klemmbar ist. Besonders bevorzugt umfassen die mehreren Klemm-, Rast- und/ oder Steckelemente zumindest ein Steckelement mit einem Federelement.

Vorzugsweise weist der Rahmen, insbesondere zumindest eine der Seiten des Rahmens und/ oder zumindest einer der abgerundeten Rahmenabschnitte, zumindest eine abgerundete Kante auf. Damit wird die Gefahr der Beschädigung eines Sterilbeutels weiter verringert. Vorzugsweise ist die abgerundete Kante an einer von dem Gitter abgewandten Seite des Rahmens vorgesehen.

Vorzugsweise ist die Trägervorrichtung aus Kunststoff hergestellt, zum Beispiel umfasst sie Polyphenylsulfon (PPSU).

Ein entsprechendes Aufbereitungsverfahren für ein dentales Instrument, bei dem das Instrument zuerst in ein Reinigungs- und/ oder Desinfektionsgerät und anschließend in eine Sterilisationsvorrichtung eingebracht wird, ist dadurch charakterisiert, dass das dentale Instrument auf einer im Vorstehenden beschriebenen Trägervorrichtung gelagert und/ oder befestigt wird, die Trägervorrichtung mit dem darauf gelagerten Instrument in das Reinigungs- und/ oder Desinfektionsgerät eingebracht und nach dem Beenden der Reinigung und/ oder Desinfektion aus dem Reinigungs- und/ oder Desinfektionsgerät entnommen und anschließend zur Sterilisation in die Sterilisationsvorrichtung eingebracht wird.

Vorzugsweise umfasst das Lagern des dentalen Instruments auf der Trägervorrichtung, dass das aufzubereitende, dentale Instrument, insbesondere ein motorischer Antrieb, in ein Steckelement mit einer Steckaufnahme eingesteckt wird und/ oder dass ein aufzubereitender Schlauch oder ein aufzubereitendes Kabel um den Wickelkörper geführt und (zumindest) an der Wickelfläche gelagert wird.

Vorzugsweise wird nach dem Einbringen der Trägervorrichtung mit dem darauf gelagerten Instrument in das Reinigungs- und/ oder Desinfektionsgerät das Instrument über einen Schlauch und/ oder einen Adapter mit einer Öffnung des Reinigungs- und/ oder Desinfektionsgeräts verbunden, von der eine Reinigungs- und/ oder Desinfektionsflüssigkeit abgegeben wird, so dass insbesondere die Flüssigkeit in das Innere des Instruments leitbar ist, um eine Innenreinigung und/ oder -desinfektion bewirken zu können. Vorzugsweise wird der Schlauch durch eine Gitteröffnung des Gitters geführt.

Vorzugsweise ist bei dem Aufbereitungsverfahren vorgesehen, dass nach dem Entnehmen der Trägervorrichtung mit dem darauf gelagerten Instrument aus dem Reinigungs- und/ oder Desinfektionsgerät (d.h. nach dem Reinigen und/ oder Desinfizieren) und vor dem Einbringen der Trägervorrichtung mit dem darauf gelagerten Instrument in die Sterilisationsvorrichtung die Trägervorrichtung mit dem darauf gelagerten Instrument in einen Sterilisationsbeutel gesteckt wird.

Vorzugsweise ist vorgesehen, dass das aufbereitete Instrument bis zur Verwendung auf der Trägervorrichtung, insbesondere in dem Sterilbeutel, gelagert wird.

Gemäß einem Ausführungsbeispiel ist eine Verwendung einer im Vorstehenden beschriebenen Trägervorrichtung in einem Aufbereitungsverfahren, insbesondere in einem im Vorstehenden beschriebenen Aufbereitungsverfahren, für ein dentales Instrument vorgesehen, bei dem die Trägervorrichtung mit dem darauf gelagerten Instrument zuerst in ein Reinigungs- und/ oder Desinfektionsgerät und anschließend in eine Sterilisationsvorrichtung eingebracht wird.

Die Erfindung wird nachfolgend anhand bevorzugter Ausführungsbeispiele und Bezug nehmend auf die beigefügten Zeichnungen erläutert. Es zeigt die
Figur 1 zeigt eine Trägervorrichtung für zumindest ein aufzubereitendes, dentales Instrument mit einer Seitenwand-Iosen Gitterplatte, von der mehrere Lagerelement für zumindest ein aufzubereitendes Instrument vorgesehen ist;
Figur 2 zeigt die mit mehreren aufzubereitenden, dentalen Instrumenten beladene Trägervorrichtung der Figur 1;
Figur 3 zeigt ein Ausführungsbeispiel eines lösbar an einer Trägervorrichtung befestigbaren Lagerelements, das ein Steckelement mit einer Steckaufnahme aufweist, in welche ein aufzubereitendes Instrument einsteckbar ist;
Figur 4 zeigt in vergrößerter Darstellung ein Ausführungsbeispiel eines Lagerelements mit einem Wickelkörper mit einer Wickelfläche, um den ein aufzubereitender Schlauch oder ein aufzubereitendes Kabel lagerbar ist.

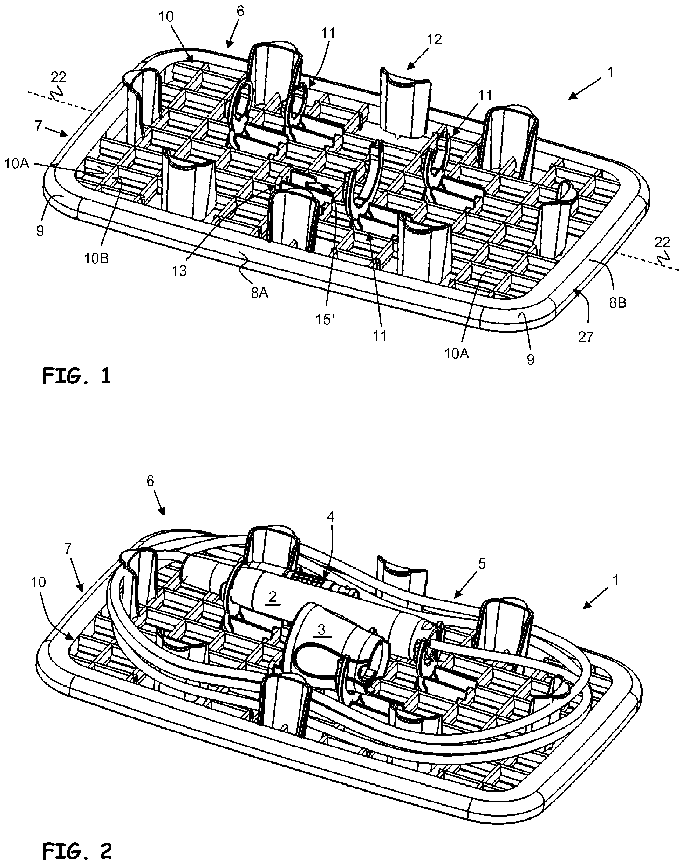

Die in den Figuren 1 und 2 dargestellte Trägervorrichtung 1 für ein oder mehrere aufzubereitende, dentale Instrumente 2 - 5 umfasst eine Seitenwand-Iose Gitterplatte 6, deren Außenumfang durch einen Rahmen 7 mit vier separaten, sich gerade erstreckenden Seiten 8A, 8B begrenzt ist. Die vier Seiten 8A, 8B sind durch abgerundete Rahmenabschnitte 9 miteinander verbunden. Die Seiten 8A, 8B des Rahmens 7 und die abgerundeten Rahmenabschnitte 9 weisen des Weiteren zumindest eine abgerundete Kante 27 auf.

Die Trägervorrichtung 1 umfasst des Weiteren ein von dem Rahmen 7 umschlossenes Gitter 10, das aus einer Vielzahl einander kreuzender Gitterstränge oder Gitterstäbe 10A, 10B besteht. Die Gitterstränge 10A verlaufen im Wesentlichen parallel zu einer Längsachse 22 der Trägervorrichtung 1 und die Gitterstränge 10B verlaufen im Wesentlichen rechtwinkelig zu der Längsachse 22. Die Gitterstränge 10A, 10B kreuzen einander im Wesentlichen im rechten Winkel.

Die Trägervorrichtung 1 und/ oder das Gitter 10 haben eine im Wesentlichen längliche, insbesondere vier- oder rechteckige Außenform. Die Trägervorrichtung 1 misst in etwa 175 cm x 275 cm.

An der Trägervorrichtung 1 ist des Weiteren zumindest ein an dem Gitter 10 befestigtes oder befestigbares und von dem Gitter 10 abstehendes Lagerelement 11 - 13 vorgesehen, an dem das aufzubereitende Instrument 2 - 5 lagerbar ist.

Die in der Figur 2 auf der Trägervorrichtung 1 gelagerten oder befestigten Instrumente umfassen beispielhaft ein dentalchirurgisches Handstück oder einen motorischen Antrieb 2 in Form eines piezoelektrischen Schwingantriebs, ein Kupplungselement 4, ein Zubehörteil in Form eines Werkzeug- oder Spitzenwechslers 3, mit dessen Hilfe Werkzeuge an dem Handstück 2 befestigbar sind sowie ein Kabel oder einen Schlauch 5, das/ der insbesondere den Antrieb 2 mit dem Kupplungselement 4 verbindet. Das Kabel 5 ist zur elektrischen Versorgung des Antriebs 2 vorgesehen.

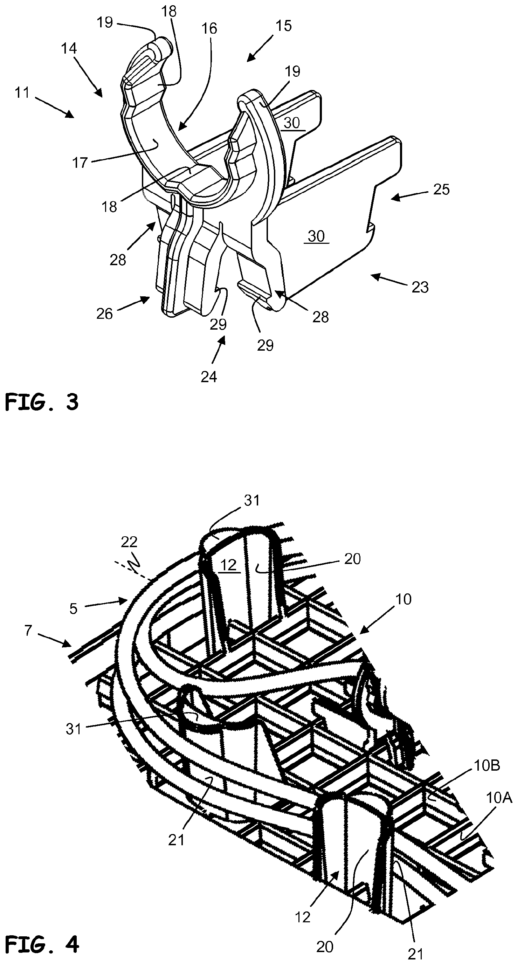

Zwecks optimaler Lagerung oder Befestigung sind für die unterschiedlichen Instrument 2 - 5 unterschiedliche Lagerelemente vorgesehen: Der motorische Antrieb 2 und das Kupplungselement 4 sind in Lagerelementen 11 gelagert oder befestigt, die ein Steckelement 14 mit einer Steckaufnahme 15 aufweisen, in welcher das aufzubereitende Instrument 2, 4 einsteckbar ist (siehe Figur 3). Das Zubehörteil 3 ist in zwei unterschiedlichen Lagerelementen 11, 13 aufgenommen, die jeweils ein Steckelement 14 mit einer Steckaufnahme 15 aufweisen, in welcher das aufzubereitende Instrument 3 einsteckbar ist. Das Kabel 5 ist um mehrere Lagerelemente 12 gewickelt, die jeweils einen Wickelkörper 20 mit einer Wickelfläche 21 aufweisen.

Das Lagerelement 11 ist mehrmals an der Trägervorrichtung 1 vorhanden, wobei insbesondere das Steckelement 14 in unterschiedlichen Abmessungen oder Dimensionen ausgebildet ist, so dass Instrumente 2 - 4 mit unterschiedlichen Außenabmessungen an der Trägervorrichtung 1 lagerbar sind. Besonders bevorzugt ist im Gegensatz dazu die Klemm-, Rast- und/ oder Steckvorrichtung 23 zur lösbaren Befestigung der Lagerelemente an dem Gitter 10 bei allen Lagerelementen 11, 13 ident.

Ein im Detail in der Figur 3 dargestelltes Lagerelement 11 umfasst ein Steckelement 14 mit einer Steckaufnahme 15, in welche das aufzubereitende Instrument 2 - 4 einsteckbar ist. Die Steckaufnahme 15 ist durch zwei gebogene Federarme 19 gebildet, deren freie Enden einander zugewandt sind. Zwischen den zwei Federarmen 19 oder durch die zwei Federarme 19 gebildet ist ein Aufnahmeraum 16 vorgesehen, in den das aufzubereitende Instrument 2 - 4 einsteckbar oder einschiebbar ist.

An einer den Aufnahmeraum 16 begrenzenden und/ oder an den Federarmen 19 vorgesehenen Wand 17 der Steckaufnahme 15 ist zumindest ein in den Aufnahmeraum 16 ragender Fortsatz 18, insbesondere eine Noppe vorgesehen, an welchem das aufzubereitende Instrument 2 - 4 lagert, so dass es von der den Aufnahmeraum 16 begrenzenden Wand 17 beabstandet ist.

Zur Befestigung des Lagerelements 11 an zumindest einem Gitterstrang 10A, 10B des Gitters 10 ist eine Klemm-, Rast- und/ oder Steckvorrichtung 23 vorgesehen. Die Klemm-, Rast- und/ oder Steckvorrichtung 23 weist mehrere Klemm-, Rast- und/ oder Steckelemente 24, 25, 28 auf, welche zumindest einen Gitterstrang 10A, 10B umgreifen oder hintergreifen. Die im Weiteren noch im Detail beschriebene Klemm-, Rast- und/ oder Steckvorrichtung 23 ist vorzugsweise für alle vom Gitter 10 lösbar ausgebildeten Lagerelemente 11, 12, 13 ident.

Ein Klemm-, Rast- und/ oder Steckelement 24 ist als Federelement 26, insbesondere als Federlasche ausgebildet. Seitlich des Federelements 26 sind zwei weitere, im Wesentlichen starre Klemm-, Rast- und/ oder Steckelemente 28 vorgesehen. Zur Befestigung des Lagerelements 11 an dem Gitter 10 ist ein Gitterstrang 10A, 10B zwischen dem Federelement 26 und den Elementen 28 aufnehmbar und wird durch Nasen 29 an den Elementen 26, 29 umgriffen.

Von jedem starren Klemm-, Rast- und/ oder Steckelement 28 erstreckt sich ein flächiges Anschlusselement 30, an denen jeweils zumindest ein weiteres Klemm-, Rast- und/ oder Steckelement 25 vorgesehen ist. Diese Steckelemente 25 sind als Rücksprünge oder Aufnahmen geformt, in denen zur Befestigung des Lagerelements 11 an dem Gitter 10 ein Gitterstrang 10A, 10B aufnehmbar ist (siehe Figur 1). Die beiden Anschlusselemente 30 sind vorzugsweise plattenförmig ausgebildet und/ oder im Wesentlichen parallel zueinander angeordnet.

Das in der Figur 1 dargestellte Lagerelement 13 lagert oder hält ein Ende des Zubehörteils 3. Dazu weist es eine Steckaufnahme 15' mit Lagerarmen auf, zwischen denen ein Aufnahmeraum für das Zubehörteil 3 vorgesehen ist. Die Lagerarme sind durch Rücksprünge in plattenförmigen Haltelementen der Steckaufnahme 15' gebildet. Das Ende des Zubehörteils 3 wird in diese Rücksprünge eingeschoben.

In der Figur 4 sind mehrere schildförmige, gebogene Lagerelemente 12 dargestellt, die vom Gitter 10 abstehen. Diese Lagerelemente 12 sind zum Lagern oder Aufwickeln des Kabels 5 vorgesehen, so dass das Kabel 5 an der Trägervorrichtung 1 gehalten wird. Diese Lagerelemente 12 umfassen jeweils einen Wickelkörper 20 mit einer Wickelfläche 21, wobei ein aufzubereitendes Kabel 5 um die Wickelkörper 20 führbar und an der Wickelfläche 21 lagerbar ist. Die Lagerelemente 12 sind unlösbar mit der Trägervorrichtung 1, insbesondere mit dem Gitter 10 verbunden.

Die Wickelfläche 21 des Wickelkörpers 20 ist im Wesentlichen rechtwinkelig zu einer Ebene angeordnet, in welcher sich das Gitter 10 der Trägervorrichtung 1 erstreckt. Am oberen, freien Ende des Wickelkörpers 20 befindet sich ein Vorsprung 31, der verhindert, dass das um den Wickelkörper 20 gewickelte Kabel 5 von dem Wickelkörper 20 gleitet.

Wie aus der Figur 4 und insbesondere auch aus den Figuren 1, 2 zu erkennen ist, sind an dem Gitter 10 mehrere Wickelkörper 20 vorgesehen, wobei die Wickelkörper 20 derart angeordnet sind, dass abwechselnd die Wickelfläche 21 eines Wickelkörper 20 der Längsachse 22 der Trägervorrichtung 1 zugewandt ist, während die Wickelfläche 21 des nächsten oder benachbarten Wickelkörpers 20 derart angeordnet ist, dass seine Wickelfläche 21 von der Längsachse 22 der Trägervorrichtung 1 abgewandt ist. Durch diese alternierende Anordnung der Wickelkörper 20 wird das Kabel 5 zwischen den Wickelkörpern 20 sicher gehalten oder geklemmt. Zwischen den am Außenrand des Gitters 10 vorgesehenen Lagerelementen 12 oder Wickelkörpern 20 sind die Lagerelemente 11, 13 angeordnet.

Die beschriebenen oder dargestellten Ausführungsbeispiele dienen insbesondere der Veranschaulichung der Erfindung. Die in einem Ausführungsbeispiel offenbarten Merkmale sind daher nicht auf dieses Ausführungsbeispiel beschränkt, sondern sind einzeln oder gemeinsam mit einem oder mehreren Merkmalen eines der anderen Ausführungsbeispiele kombinierbar.

## Patentansprüche

1. Trägervorrichtung (1) für zumindest ein aufzubereitendes, dentales Instrument (2 - 5), **gekennzeichnet durch**: eine Seitenwand-Iose Gitterplatte (6), deren Außenumfang durch einen Rahmen (7) mit vier separaten, sich gerade erstreckenden Seiten (8A, 8B) begrenzt ist, wobei die vier Seiten (8A, 8B) des Rahmens (7) durch abgerundete Rahmenabschnitte (9) miteinander verbunden sind, ein von dem Rahmen (7) umschlossenes Gitter (10) und zumindest ein an dem Gitter (10) befestigtes oder befestigbares und von dem Gitter (10) abstehendes Lagerelement (11 - 13), an dem das aufzubereitende Instrument (2 - 5) lagerbar ist.

2. Trägervorrichtung (1) nach Anspruch 1, **dadurch gekennzeichnet, dass** das zumindest eine Lagerelement (11 - 13) ein Steckelement (14) mit einer Steckaufnahme (15, 15') aufweist, in welche das aufzubereitende Instrument (2 - 5) einsteckbar ist.

3. Trägervorrichtung (1) nach Anspruch 2, **dadurch gekennzeichnet, dass** die Steckaufnahme (15) einen Aufnahmeraum (16) definiert, in dem zumindest ein Teil des aufzubereitenden Instruments (2 - 5) aufnehmbar ist, wobei an einer den Aufnahmeraum (16) begrenzenden Wand (17) der Steckaufnahme (15) zumindest ein in den Aufnahmeraum (16) ragender Fortsatz (18), insbesondere eine Noppe vorgesehen ist, an welchem das aufzubereitende Instrument (2 - 5) lagert, so dass es von der den Aufnahmeraum (16) begrenzenden Wand (17) beabstandet ist.

4. Trägervorrichtung (1) nach Anspruch 2 oder 3, **dadurch gekennzeichnet, dass** die Steckaufnahme (15, 15') zwei Federarme (19) oder zwei Lagerarme aufweist, zwischen denen der Aufnahmeraum (16) vorgesehen ist.

5. Trägervorrichtung (1) nach Anspruch 1, **dadurch gekennzeichnet, dass** das zumindest eine Lagerelement (11 - 13) einen vom Gitter (10) abstehenden, insbesondere schildförmigen, Wickelkörper (20) mit einer Wickelfläche (21) aufweist, wobei ein aufzubereitender Schlauch oder ein aufzubereitendes Kabel (5) um den Wickelkörper (20) führbar und an der Wickelfläche (21) lagerbar ist.

6. Trägervorrichtung (1) nach Anspruch 5, **dadurch gekennzeichnet, dass** die Wickelfläche (21) des Wickelkörpers (20) gebogen oder konvex ausgebildet ist oder einen an dem Wickelkörper (20) vorgesehenen gebogenen oder konvexen Überstand aufweist, so dass eine Kontaktfläche zwischen dem aufzubereitenden Schlauch oder Kabel (5) und der Wickelfläche (21) möglichst gering ist.

7. Trägervorrichtung (1) nach Anspruch 5 oder 6, **dadurch gekennzeichnet, dass** die Wickelfläche (21) des Wickelkörpers (20) im Wesentlichen rechtwinkelig zu einer Ebene angeordnet ist, in welcher sich das Gitter (10) der Trägervorrichtung (1) erstreckt.

8. Trägervorrichtung (1) nach Anspruch 5, 6 oder 7, **gekennzeichnet durch** mehrere an dem Gitter (10) vorgesehene Wickelkörper (20), wobei zumindest einer dieser mehreren Wickelkörper (20) derart angeordnet oder anordenbar ist, dass seine Wickelfläche (21) einer Längsachse (22) der Trägervorrichtung (1) zugewandt ist, während zumindest ein anderer dieser mehreren Wickelkörper (20) derart angeordnet oder anordenbar ist, dass seine Wickelfläche (21) von der Längsachse (22) der Trägervorrichtung (1) abgewandt ist.

9. Trägervorrichtung (1) nach einem der Ansprüche 2 - 8, **gekennzeichnet durch** zumindest ein Lagerelement (11, 13), das ein Steckelement (14) mit einer Steckaufnahme (15, 15') aufweist, in welche ein aufzubereitendes, dentales Instrument (2 - 5), insbesondere ein motorischer Antrieb (2), einsteckbar ist und durch mehrere Lagerelemente (12), die jeweils einen Wickelkörper (20) mit einer Wickelfläche (21) aufweisen, so dass ein aufzubereitender Schlauch oder ein aufzubereitendes Kabel (5), insbesondere des in dem Steckelement (14) eingesteckten, aufzubereitenden Instruments (2, 4), um die Wickelkörper (20) führbar und an ihren Wickelflächen (21) lagerbar ist.

10. Trägervorrichtung (1) nach Anspruch 9, **dadurch gekennzeichnet, dass** das zumindest eine Lagerelement (11, 13), das ein Steckelement (14) mit einer Steckaufnahme (15, 15') aufweist, und die mehreren Lagerelemente (12), die jeweils einen Wickelkörper (20) mit einer Wickelfläche (21) aufweisen, derart auf der Trägervorrichtung (1) angeordnet sind, dass der aufzubereitende Schlauch oder das aufzubereitende Kabel (5) nicht von dem in dem Steckelement (14) eingesteckten, aufzubereitenden Instrument (2 - 5) gelöst werden muss.

11. Trägervorrichtung (1) nach einem der vorstehenden Ansprüche, **dadurch gekennzeichnet, dass**
das zumindest eine Lagerelement (11 - 13) zur Befestigung an zumindest einem Gitterstrang (10A, 10B) des Gitters (10) eine Klemm-, Rast- und/ oder Steckvorrichtung (23) aufweist.

12. Trägervorrichtung (1) nach Anspruch 11, **dadurch gekennzeichnet, dass** die Klemm-, Rast- und/ oder Steckvorrichtung (23) zumindest ein Klemm-, Rast- und/ oder Steckelement (24, 25) aufweist, das zumindest einen Gitterstrang (10A, 10B) des Gitters (10) umgreift oder hintergreift, und/ oder dass das zumindest eine Klemm-, Rast- und/ oder Steckelement (24) ein Federelement (26) aufweist.

13. Trägervorrichtung (1) nach einem der vorstehenden Ansprüche, **dadurch gekennzeichnet, dass**
der Rahmen (7), insbesondere zumindest eine der Seiten (8A, 8B) des Rahmens (7) und/ oder zumindest einer der abgerundeten Rahmenabschnitte (9), zumindest eine abgerundete Kante (27) aufweist.

14. Aufbereitungsverfahren für ein dentales Instrument (2 - 5), bei dem das Instrument (2 - 5) zuerst in ein Reinigungs- und/ oder Desinfektionsgerät und anschließend in eine Sterilisationsvorrichtung eingebracht wird, **dadurch gekennzeichnet, dass** das dentale Instrument (2 - 5) auf einer Trägervorrichtung (1) nach einem der vorstehenden Ansprüche gelagert wird, die Trägervorrichtung (1) mit dem darauf gelagerten Instrument (2 - 5) in das Reinigungs- und/ oder Desinfektionsgerät eingebracht und nach dem Beenden der Reinigung und/ oder Desinfektion aus dem Reinigungs- und/ oder Desinfektionsgerät entnommen und anschließend zur Sterilisation in die Sterilisationsvorrichtung eingebracht wird.

15. Verwendung einer Trägervorrichtung (1) nach einem der vorstehenden Ansprüche 1 - 13 in einem Aufbereitungsverfahren, insbesondere in einem Aufbereitungsverfahren nach Anspruch 14, für ein dentales Instrument (2 - 5), bei dem die Trägervorrichtung (1) mit dem darauf gelagerten Instrument (2 - 5) zuerst in ein Reinigungs- und/ oder Desinfektionsgerät und anschließend in eine Sterilisationsvorrichtung eingebracht wird.

## Geänderte Patentansprüche

### Geänderte Patentansprüche gemäss Regel 137(2) EPÜ.

1. Trägervorrichtung (1) für zumindest ein aufzubereitendes, dentales Instrument (2 - 5), **gekennzeichnet durch**: eine Seitenwand-lose, ebene Gitterplatte (6), deren Außenumfang durch einen Rahmen (7) mit vier separaten, sich gerade erstreckenden Seiten (8A, 8B) begrenzt ist, wobei die vier Seiten (8A, 8B) des Rahmens (7) durch abgerundete Rahmenabschnitte (9) miteinander verbunden sind, ein von dem Rahmen (7) umschlossenes Gitter (10) und zumindest ein an dem Gitter (10) befestigtes oder befestigbares und von dem Gitter (10) abstehendes Lagerelement (11 - 13), an dem das aufzubereitende Instrument (2 - 5) lagerbar ist, wobei sich das Gitter (10), die vier Seiten (8A, 8B) des Rahmens (7) und die abgerundeten Rahmenabschnitten (9) der Seitenwand-losen, ebenen Gitterplatte (6) alle in derselben Ebene erstrecken.

2. Trägervorrichtung (1) nach Anspruch 1, **dadurch gekennzeichnet, dass** das zumindest eine Lagerelement (11 - 13) ein Steckelement (14) mit einer Steckaufnahme (15, 15') aufweist, in welche das aufzubereitende Instrument (2 - 5) einsteckbar ist.

3. Trägervorrichtung (1) nach Anspruch 2, **dadurch gekennzeichnet, dass** die Steckaufnahme (15) einen Aufnahmeraum (16) definiert, in dem zumindest ein Teil des aufzubereitenden Instruments (2 - 5) aufnehmbar ist, wobei an einer den Aufnahmeraum (16) begrenzenden Wand (17) der Steckaufnahme (15) zumindest ein in den Aufnahmeraum (16) ragender Fortsatz (18), insbesondere eine Noppe vorgesehen ist, an welchem das aufzubereitende Instrument (2 - 5) lagert, so dass es von der den Aufnahmeraum (16) begrenzenden Wand (17) beabstandet ist.

4. Trägervorrichtung (1) nach Anspruch 2 oder 3, **dadurch gekennzeichnet, dass** die Steckaufnahme (15, 15') zwei Federarme (19) oder zwei Lagerarme aufweist, zwischen denen der Aufnahmeraum (16) vorgesehen ist.

5. Trägervorrichtung (1) nach Anspruch 1, **dadurch gekennzeichnet, dass** das zumindest eine Lagerelement (11 - 13) einen vom Gitter (10) abstehenden, insbesondere schildförmigen, Wickelkörper (20) mit einer Wickelfläche (21) aufweist, wobei ein aufzubereitender Schlauch oder ein aufzubereitendes Kabel (5) um den Wickelkörper (20) führbar und an der Wickelfläche (21) lagerbar ist.

6. Trägervorrichtung (1) nach Anspruch 5, **dadurch gekennzeichnet, dass** die Wickelfläche (21) des Wickelkörpers (20) gebogen oder konvex ausgebildet ist oder einen an dem Wickelkörper (20) vorgesehenen gebogenen oder konvexen Überstand aufweist, so dass eine Kontaktfläche zwischen dem aufzubereitenden Schlauch oder Kabel (5) und der Wickelfläche (21) möglichst gering ist.

7. Trägervorrichtung (1) nach Anspruch 5 oder 6, **dadurch gekennzeichnet, dass** die Wickelfläche (21) des Wickelkörpers (20) im Wesentlichen rechtwinkelig zu einer Ebene angeordnet ist, in welcher sich das Gitter (10) der Trägervorrichtung (1) erstreckt.

8. Trägervorrichtung (1) nach Anspruch 5, 6 oder 7, **gekennzeichnet durch** mehrere an dem Gitter (10) vorgesehene Wickelkörper (20), wobei zumindest einer dieser mehreren Wickelkörper (20) derart angeordnet oder anordenbar ist, dass seine Wickelfläche (21) einer Längsachse (22) der Trägervorrichtung (1) zugewandt ist, während zumindest ein anderer dieser mehreren Wickelkörper (20) derart angeordnet oder anordenbar ist, dass seine Wickelfläche (21) von der Längsachse (22) der Trägervorrichtung (1) abgewandt ist.

9. Trägervorrichtung (1) nach einem der Ansprüche 2 - 8, **gekennzeichnet durch** zumindest ein Lagerelement (11, 13), das ein Steckelement (14) mit einer Steckaufnahme (15, 15') aufweist, in welche ein aufzubereitendes, dentales Instrument (2 - 5), insbesondere ein motorischer Antrieb (2), einsteckbar ist und durch mehrere Lagerelemente (12), die jeweils einen Wickelkörper (20) mit einer Wickelfläche (21) aufweisen, so dass ein aufzubereitender Schlauch oder ein aufzubereitendes Kabel (5), insbesondere des in dem Steckelement (14) eingesteckten, aufzubereitenden Instruments (2, 4), um die Wickelkörper (20) führbar und an ihren Wickelflächen (21) lagerbar ist.

10. Trägervorrichtung (1) nach Anspruch 9, **dadurch gekennzeichnet, dass** die mehreren Lagerelemente (12), die jeweils einen Wickelkörper (20) mit einer Wickelfläche (21) aufweisen, das zumindest eine Lagerelement (11, 13), das ein Steckelement (14) mit einer Steckaufnahme (15, 15') aufweist, umgeben, so dass der aufzubereitende Schlauch oder das aufzubereitende Kabel (5) nicht von dem in dem Steckelement (14) eingesteckten, aufzubereitenden Instrument (2 - 5) gelöst werden muss.

11. Trägervorrichtung (1) nach einem der vorstehenden Ansprüche, **dadurch gekennzeichnet, dass**
das zumindest eine Lagerelement (11 - 13) zur Befestigung an zumindest einem Gitterstrang (10A, 10B) des Gitters (10) eine Klemm-, Rast- und/ oder Steckvorrichtung (23) aufweist.

12. Trägervorrichtung (1) nach Anspruch 11, **dadurch gekennzeichnet, dass** die Klemm-, Rast- und/ oder Steckvorrichtung (23) zumindest ein Klemm-, Rast- und/ oder Steckelement (24, 25) aufweist, das zumindest einen Gitterstrang (10A, 10B) des Gitters (10) umgreift oder hintergreift, und/ oder dass das zumindest eine Klemm-, Rast- und/ oder Steckelement (24) ein Federelement (26) aufweist.

13. Trägervorrichtung (1) nach einem der vorstehenden Ansprüche, **dadurch gekennzeichnet, dass**
der Rahmen (7), insbesondere zumindest eine der Seiten (8A, 8B) des Rahmens (7) und/ oder zumindest einer der abgerundeten Rahmenabschnitte (9), zumindest eine abgerundete Kante (27) aufweist.

14. Aufbereitungsverfahren für ein dentales Instrument (2 - 5), bei dem das Instrument (2 - 5) zuerst in ein Reinigungs- und/ oder Desinfektionsgerät und anschließend in eine Sterilisationsvorrichtung eingebracht wird, **dadurch gekennzeichnet, dass** das dentale Instrument (2 - 5) auf einer Trägervorrichtung (1) nach einem der vorstehenden Ansprüche gelagert wird, die Trägervorrichtung (1) mit dem darauf gelagerten Instrument (2 - 5) in das Reinigungs- und/ oder Desinfektionsgerät eingebracht und nach dem Beenden der Reinigung und/ oder Desinfektion aus dem Reinigungs- und/ oder Desinfektionsgerät entnommen und anschließend zur Sterilisation in die Sterilisationsvorrichtung eingebracht wird.

15. Verwendung einer Trägervorrichtung (1) nach einem der vorstehenden Ansprüche 1 - 13 in einem Aufbereitungsverfahren, insbesondere in einem Aufbereitungsverfahren nach Anspruch 14, für ein dentales Instrument (2 - 5), bei dem die Trägervorrichtung (1) mit dem darauf gelagerten Instrument (2 - 5) zuerst in ein Reinigungs- und/ oder Desinfektionsgerät und anschließend in eine Sterilisationsvorrichtung eingebracht wird.
